# EUROPEAN PATENT APPLICATION

(11) **EP 4 112 046 A1**
(43) Date of publication of application: **04.01.2023**
(21) Application number: 22179641.0
(22) Date of filing: 17.06.2022
(51) Int. Cl.: A61K 9/16, A61K 31/4245, A61P 3/10

(54) **PHARMACEUTICAL COMPOSITION AND USE THEREOF**

(30) Priority: 02.07.2021 US 202117366313
(71) Applicant: Alphala Co., Ltd., Taipei City 103 (TW)
(72) Inventor: HONG, Chi-Jen, 103 Taipei City (TW); CHEN, Yen-Fu, 103 Taipei City (TW); CHUNG, Cheng-Ho, 103 Taipei City (TW)
(74) Representative: van Dam, Vincent

(57) **Abstract**

Disclosed is a pharmaceutical composition comprising: an active ingredient of the following formula (I) or a pharmaceutically acceptable salt or solvate thereof present in an amount of 8 wt% to 30 wt%: 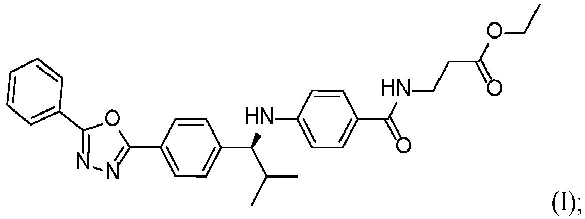 and two or more excipients at least including a dispersant and a solubilizer present in an amount of 70 wt% to 92 wt%.. Also disclosed are methods for reducing the glycemic level and treating disorders associated with glucagon with the aforesaid pharmaceutical composition.

## Description

### FIELD OF THE DISCLOSURE

The present disclosure relates to a pharmaceutical composition comprising a glucagon receptor antagonist, and the use thereof.

### BACKGROUND

ALP001E is a glucagon receptor antagonist and used for treatment of diabetes mellitus. ALP001E is a Biopharmaceutical Classification System (BCS) Class II drug which indicates poor aqueous solubility but good membrane permeability. It is stable in the solid state and not sensitive to light irradiation. However, ALP001E predominantly undergoes degradation by hydrolytic pathways in the presence of moisture. It is also sensitive to acid or basic environments.

Because of these physicochemical and biopharmaceutical properties of ALP001E, several attempts have been made to provide compositions of ALP001E that are stable and provide desirable *in vitro* release and bioavailability.

Thus, it is desirable to provide a novel pharmaceutical composition comprising ALP001E, so that ALP001E can be applied to clinical use.

### SUMMARY

The present disclosure relates to a novel pharmaceutical composition comprising a glucagon receptor antagonist, ALP001E, with good stability and desired *in vitro* or *in vivo* performance.

An aspect of the present disclosure is drawn to a pharmaceutical composition, comprising: an active ingredient of the following formula (I) or a pharmaceutically acceptable salt or solvate thereof present in an amount of 8 wt% to 30 wt%: 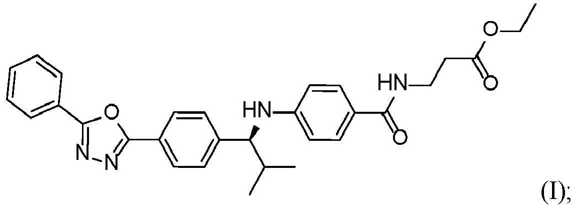 and
two or more excipients at least including a dispersant and a solubilizer present in an amount of 70 wt% to 92 wt%.

In addition to the active ingredient of the formula (I) described above, their pharmaceutically acceptable salts and solvates, where applicable, are also covered by the present disclosure. A salt can be formed between an anion and a positively charged group (e.g., amino) on a compound. Examples of a suitable anion include chloride, bromide, iodide, sulfate, nitrate, phosphate, citrate, methanesulfonate, trifluoroacetate, acetate, malate, tosylate, tartrate, fumurate, glutamate, glucuronate, lactate, glutarate, and maleate. A salt can also be formed between a cation and a negatively charged group. Examples of a suitable cation include sodium ion, potassium ion, magnesium ion, calcium ion, and an ammonium cation such as tetramethylammonium ion. A salt further includes those containing quaternary nitrogen atoms. A solvate refers to a complex formed between an active compound and a pharmaceutically acceptable solvent. Examples of a pharmaceutically acceptable solvent include water, ethanol, isopropanol, ethyl acetate, acetic acid, and ethanolamine.

Another aspect of the present disclosure is drawn to a method for reducing the glycemic level in a subject, comprising administering to the subject in need thereof the aforesaid pharmaceutical composition.

A further aspect of the present disclosure is drawn to a method of treating disorders associated with glucagon, comprising administering to the subject in need thereof the aforesaid pharmaceutical composition.

The pharmaceutical composition of the present invention can be administered to a subject orally, parenterally, by inhalation spray, topically, rectally, nasally, buccally, or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques.

In the present disclosure, the pharmaceutical composition can be an oral pharmaceutical composition, which can be any orally acceptable dosage form including capsules, tablets, emulsions and aqueous suspensions, dispersions, and solutions. Oral solid dosage forms can be prepared by spray dried techniques; hot melt extrusion strategy, micronization, and nano milling technologies. Althernatively, the pharmaceutical composition can be a nasal aerosol or inhalation composition, which can be prepared according to techniques well known in the art of pharmaceutical formulation. Further alternatively, the pharmaceutical composition of the present disclosure can also be administered in the form of suppositories for rectal administration.

The excipient in the pharmaceutical composition must be "acceptable" in the sense that it is compatible with the active ingredient of the composition (and preferably, capable of stabilizing the active ingredient) and not deleterious to the subject to be treated.

The term "treating", "treat" or "treatment" refers to application or administration of the active ingredient to a subject with the purpose to cure, alleviate, relieve, alter, remedy, improve, or affect the disease, the symptom, or the predisposition. "An effective amount" refers to the amount of the active ingredient which is required to confer the desired effect on the subject. Effective amounts vary, as recognized by those skilled in the art, depending on route of administration, excipient usage, and the possibility of co-usage with other therapeutic treatments such as use of other active agents.

The term "weight percentages" (i.e., "% by weight" and "wt %" and w/w) referenced herein, unless otherwise indicated, are based on the total weight of the pharmaceutical composition unless specified otherwise.

The details of one or more embodiments of the disclosure are set forth in the description below. Other features, objects, and advantages of the disclosure will be apparent from the description and from the claims.

### DETAILED DESCRIPTION

Disclosed in detail below is a pharmaceutical composition, comprising: an active ingredient of the following formula (I) or a pharmaceutically acceptable salt or solvate thereof present in an amount of 8 wt% to 30 wt%: 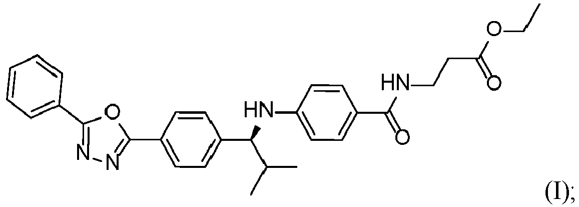 and
two or more excipients at least including a dispersant and a solubilizer present in an amount of 70 wt% to 92 wt%.

The present disclosure provides a novel pharmaceutical composition of ALP001E, which comprises two or more excipients at least including a dispersant and a solubilizer. The pharmaceutical composition provided by the present disclosure is chemically and polymorphically stable. In addition, the pharmaceutical composition of the present disclosure exhibits desired *in vitro* and *in vivo* performance and can be prepared by simple, ono-tedious and cost-effective process, such as spray dry method. Furthermore, the dry powders of the pharmaceutical composition provided by the present disclosure have acceptable solubility and increased oral absorption by a specific formulation ratio and process parameters, which can carry out the possibility of entering the GMP pharmaceutical industry.

In the pharmaceutical composition of the present disclosure, the excipients may selectively further comprise: a binder, a diluent, a disintegrant, an emulsifier, a filler, a glidant, a lubricant, a plasticizer, a thickener or a wetting agent.

Examples of the binder may include celluloses (such as ethyl cellulose, cellulose acetate, carboxymethyl cellulose calcium, sodium carboxymethyl cellulose, methyl cellulose, hydroxyethylcellulose (HEC), hydroxypropylcellulose (HPC), hydroxypropyl methyl cellulose (HPMC)), cross-linked polymers (such as soluplus), gelatin, microcrystalline celluloses, natural and synthetic gums (such as acacia, alginic acid, alginates, extract of Irish moss, Panwar gum, ghatti gum, mucilage of isabgol husks, carboxymethylcellulose, methylcellulose, polyvinylpyrrolidone (PVP), Veegum, larch arabogalactan, powdered tragacanth, and guar gum), starches (such as corn starch, potato starch, pre-gelatinized starch and partially pre-gelatinized maize starch), sugars (such as sucrose, glucose, dextrose, molasses, and lactose) or a combination thereof; but the present disclosure is not limited thereto.

Examples of the diluent may include calcium sulfate, cellulose, dibasic calcium phosphate, dry starch, inositol, kaolin, lactose, mannitol, microcrystalline celloluse, powdered sugar, sodium chloride, sorbitol, starch (such as directly compressed starch and hydrolyzed starch), sucrose, or a combination thereof; but the present disclosure is not limited thereto.

Examples of the disintegrant may include agar, alginic acid, alginate, aligns, bentonite, calcium carbonate, cation-exchange resins, celluloses (such as methylcellulose and carboxymethylcellulose), citrus pulp, clays, cross-linked celluloses (such as croscarmellose and croscarmellose sodium), cross-linked polymers (such as crospovidone), cross-linked starches, gums (such as guar gum and Veegum HV), microcrystalline cellulose (such as various types of sodium starch glycolate), natural sponge, polacrilin potassium, starches (such as corn starch, potato starch, tapioca starch, and pre-gelatinized starch), wood products or a combination thereof; but the present disclosure is not limited thereto.

Examples of the dispersant may include acacia, hydroxypropyl methylcellulose, hypromellose acetate succinate (HPMCAS), pectin, polyvinylpyrolidone, sodium carbomethylcellulose, sodium carboxymethylcellulose, tragacanth, Veegum or a combination thereof; but the present disclosure is not limited thereto.

Examples of the emulsifier may include acacia, bentonite, gelatin, tragacanth, triethanolamine oleate, Tween 20, Tween 80 or a combination thereof; but the present disclosure is not limited thereto.

Examples of the filler may include calcium carbonate, dextrates, kaolin, mannitol, microcrystalline cellulose, pre-gelatinized starch, powdered cellulose, silicic acid, sorbitol, starch, talc or a combination thereof; but the present disclosure is not limited thereto.

Examples of the glidant (also named as the flowing agent) may include corn starch, talc, solicon dioxide, colloidal silicone dioxide, or a combination thereof; but the present disclosure is not limited thereto.

Examples of the lubricant may include agar, calcium stearate, ethyl laureate, ethyl oleate, glycerin, glycols (such as glycerol behenate and polyethylene glycol (PEG)), hydrogenated vegetable oil (such as peanut oil, cottonseed oil, sunflower oil, sesame oil, olive oil, corn oil, and soybean oil), light mineral oil, lycopodium, magnesium stearate, mannitol, mineral oil, silica or silica gels, sodium lauryl sulfate, sodium stearyl fumarate, sorbitol, stearic acid, talc, wax, zinc stearate or a combination thereof; but the present disclosure is not limited thereto.

Examples of the plasticizer may include castor oil, citrate esters (such as triethyl citrate, tributyl citrate, acetyl triethyl citrate and acetyl tributyl citrate), fatty acid esters (such as butyl stearate, glycerol monostearate and stearyl alcohol), glycol derivatives (such as polyethylene glycol and propylene glycol), mineral oil, phthalate esters (such as diethyl phthalate, dibutyl phthalate and dioctyl phosphate), sebacate esters (such as dibutyl sebacate), triacetin, vitamin E TPGS (D-α-tocopheryl polyethylene glycol 1000 succinate) or a combination thereof; but the present disclosure is not limited thereto.

Examples of the solubilizer may include citric acid, tartaric acid, benzoic acid, polyethylene glycol, ethanol, propylene glycol, glycerin, n-methyl 2-pyrrolidone, dimethyl acetamide, beeswax, d-α tocopherol, oleic acid, mono and di glycerides, cremphor, Tween 20, sorbitan monooleate, peppermint oil, polysorbate, peanut oil, corn oil, soybean oil, sesame oil, olive oil, cotton seed oil, α-cyclodextrins, β-cyclodextrins, γ-cyclodextrins, sulfobutylether-cyclodextrin, hydroxypropyl-cyclodextrin, glycerol, choline, distearoyl phosphatidylglycerol (DSPG), dimyristoylphosphatidylcholine (DMPC), dimyristoylphosphatidylglycerol (DMPG), Macrogol 15 Hydroxystearate or a combination thereof; but the present disclosure is not limited thereto.

Examples of the thickener may include acacia, agar, alamic acid, alginic acid, aluminum monostearate, attapulgite, bentonite, carbomer, carbomer copolymer, carbomer homopolymer, carbomer interpolymer, carboxymethylcellulose calcium, carboxymethylcellulose sodium, carrageenan, cellulose, dextrin, gelatin, gum (such as gellan gum, guar gum and xanthan gum), hydroxyethyl cellulose, hydroxypropyl cellulose, hypromellose, magnesium aluminum silicate, maltodextrin, methylcellulose, microcrystalline cellulose, pectin, polyethylene oxide, polyvinyl alcohol, povidone, propylene glycol aliginate, silicon dioxide, colloidal silicon dioxide, sodium alginate, starch (including corn starch, potato starch, tapioca starch and wheat starch), tragacanth or a combination thereof; but the present disclosure is not limited thereto.

Examples of the wetting agent may include diethylene glycol monolaurate, propylene glycol monostearate, polyoxyethylene lauryl ether, sorbitan monooleate, sodium lauryl sulfate (SLS), sodium stearyl fumarate (SSF), Tween 20, Tween 80 or a combination thereof; but the present disclosure is not limited thereto.

In the present disclosure, the pharmaceutical composition comprises two excipients including the dispersant and the solubilizer.

In the present disclosure, the active ingredient of the formula (I) or the pharmaceutically acceptable salt or solvate thereof may be present in an amount of 8 wt% to 30 wt%, 9 wt% to 30 wt%, 9 wt% to 29 wt%, 9 wt% to 28 wt%, 10 wt% to 28 wt%, 11 wt% to 28 wt%, 12 wt% to 28 wt%, 13 wt% to 28 wt%, 13 wt% to 27 wt%, 14 wt% to 27 wt%, 14.5 wt to 27 wt%, 14.5 wt% to 26 wt%, 14.5 wt% to 25 wt%, 14.5 wt% to 24 wt%, 14.5 wt% to 23 wt%, 14.5 wt% to 22 wt%, 14.5 wt% to 21 wt%, 14.5 wt% to 20 wt%, 14.5 wt% to 19 wt%, 15 wt% to 19 wt%, 15 wt% to 18.5 wt% or 15.5 wt% to 18.5 wt%.

In the present disclosure, the dispersant may be present in an amount of 40 wt% to 88 wt%, 45 wt% to 88 wt%, 50 wt% to 88 wt%, 55 wt% to 88 wt%, 60 wt% to 88 wt%, 60 wt% to 87 wt%, 60 wt% to 86 wt%, 60 wt% to 85 wt%, 60 wt% to 84 wt%, 60 wt% to 83 wt%, 60 wt% to 82 wt%, 60 wt% to 81 wt%, 60 wt% to 80 wt%, 61 wt% to 80 wt%, 62 wt% to 80 wt%, 63 wt% to 80 wt%, 64 wt% to 80 wt%, 65 wt% to 80 wt%, 66 wt% to 80 wt%, 67 wt% to 80 wt%, 68 wt% to 80 wt%, 69 wt% to 80 wt%, 70 wt% to 80 wt%, 71 wt% to 80 wt%, 71 wt% to 79 wt%, 72 wt% to 79 wt%, 72 wt% to 78 wt%, 73 wt% to 78 wt% or 73 wt% to 77 wt%.

In the present disclosure, the solubilizer may be present in an amount of 2 wt% to 20 wt%, 2 wt% to 19 wt%, 2 wt% to 18 wt%, 2 wt% to 17 wt%, 2 wt% to 16 wt%, 2 wt% to 15 wt%, 3 wt% to 15 wt%, 3 wt% to 14 wt%, 4 wt% to 14 wt%, 4 wt% to 13 wt%, 5 wt% to 13 wt%, 5 wt% to 12 wt%, 5 wt% to 11 wt%, 5 wt% to 10 wt%, 6 wt% to 10 wt%, 6 wt% to 9 wt% or 7 wt% to 9 wt%.

In the present disclosure, a weight ratio of the dispersant to the solubilizer may be ranged from 3.0 to 20.0, 3.5 to 20.0, 4.0 to 20.0, 4.5 to 20.0, 4.5 to 19.5, 4.5 to 19.0, 4.5 to 18.5, 4.5 to 18.0, 4.5 to 17.5, 4.5 to 17.0, 4.5 to 16.5, 4.5 to 16.0, 4.5 to 15.5, 4.5 to 15.0, 4.5 to 14.5, 4.5 to 14.0, 4.5 to 13.5, 4.5 to 13.0, 4.5 to 12.5, 5.0 to 12.5, 5.0 to 12.0, 5.5 to 12.0, 5.5 to 11.5, 6.0 to 11.5, 6.0 to 11.0, 6.5 to 11.0, 7.0 to 11.0, 7.5 to 11.0, 8.0 to 11.0, 8.5 to 11.0 or 8.5 to 10.5. When this weight ratio is in the aforesaid range, the pharmaceutical composition of the present disclosure have an enhance effect for improving of solubility or oral bioavailability *in vivo.*

In one embodiment of the prenset disclosure, the dispersant may comprise hypromellose acetate succinate. However, the present disclosure is not limited thereto, and any other dispersant illustrated above may be used in the pharmaceutical composition of the present disclosure according to the need.

In one embodiment of the present disclosure, the solubilizer may comprise Macrogol 15 Hydroxystearate. However, the present disclosure is not limited thereto, and any other solubilizer illustrated above may be used in the pharmaceutical composition of the present disclosure according to the need.

In a first embodiment of the present disclosure, the active ingredient of the formula (I) or the pharmaceutically acceptable salt or solvate thereof may be present in an amount of 8 wt% to 30 wt%; the dispersant may be present in an amount of 40 wt% to 88 wt%; and the solubilizer may be present in an amount of 2 wt% to 20 wt%.

In a second embodiment of the present disclosure, the active ingredient of the formula (I) or the pharmaceutically acceptable salt or solvate thereof may be present in an amount of 9 wt% to 28 wt%; the dispersant may be present in an amount of 60 wt% to 87 wt%; and the solubilizer may be present in an amount of 3 wt% to 15 wt%.

In a third embodiment of the present disclosure, the active ingredient of the formula (I) or the pharmaceutically acceptable salt or solvate thereof may be present in an amount of 10 wt% to 28 wt%; the dispersant may be present in an amount of 60 wt% to 85 wt%; and the solubilizer may be present in an amount of 5 wt% to 13 wt%.

In a fourth embodiment of the present disclosure, the active ingredient of the formula (I) or the pharmaceutically acceptable salt or solvate thereof may be present in an amount of 14.5 wt% to 20 wt%; the dispersant may be present in an amount of 70 wt% to 80 wt%; and the solubilizer may be present in an amount of 5 wt% to 10 wt%.

In any of the aforesaid first to fourth embodiments, the dispersant may comprise hypromellose acetate succinate.

In any of the aforesaid first to fourth embodiments, the solubilizer may comprise Macrogol 15 Hydroxystearate.

In the aforesaid embodiments, the pharmaceutical composition comprises an effective amount of the active ingredient. The effective amout may be, for example, in a range from 0.01 to 10 mg, 0.1 to 10 mg, 1 to 10 mg, 1 to 8 mg, 2 to 8 mg, or 2 to 7 mg per kg of the subject in need thereof. However, the present disclosure is not limited thereto, and the effective amout may be adjusted according to subject, route of administration, excipient usage, and the possibility of co-usage with other therapeutic treatments such as use of other active agents.

In the aforesaid embodiments, the pharmaceutical composition can be formulated into powders. Herein, the method for preparing the powders of the pharmaceutical composition may be a spray drying method, but the present disclosure is not limited thereto.

When the powders of the pharmaceutical composition are prepared by the spray drying method, the inlet temperature may be ranged from 69°C to 101°C or 70°C to 85°C. The outlet temperature may be ranged from 42°C to 52°C. The atomization pressure may be ranged from 0.15 MPa to 0.22 Mpa. The blower may be ranged from scale 5 to scale 7. The air volume may be set at a range from 0.20 m³/min to 0.60 m³/min, for example 0.40 m³/min. The oxygen content may be set at <3%. However, the present disclosure is not limited thereto. The parameters for the spray drying method can be varied according to the diameters of the desired powders or the components of the pharmaceutical compositions.

In the present disclosure, the water poorly soluble active ingredient (ALP001E) can be prepared by the solvent spray drying method when the active ingredient is formulated into the pharmaceutical composition of the present disclosure. The powders of the pharmaceutical composition of the present disclosure show improved solubility or increased bioavailability. In addition, the specific formulation and process parameter of the pharmaceutical composition of the present disclosure can be implemented for pharmaceutical technology with process and operation advantages. For example, the process using the inlet temperature with a gentle range from 69°C to 101°C can rapidly produce spray dry powders and be easy to operate.

In the aforesaid embodiments, the powders of the pharmaceutical composition can be placed inside a capsule. The material of the capsule can be, for example, gelatin.

In the aforesaid embodiemnts, the powders of the pharmaceutical compostion can be compressed to form a tablet, a granule or a particle. When the pharmaceutical composition is formulated into the tablet, the granule or the particle, the pharmaceutical composition may further comprise a coating layer, wherein the active ingredient and the excipients are included whitin the coating layer.

In the aforesaid embodiments, the pharmaceutical composition can be an oral pharmaceutical composition, which can be formulated into powders, capsules, tablets or granules.

In the aforesaid embodiments, the pharmaceutical composition can be used in reducing the glycemic level in a subject thereof.

In the aforesaid embodiments, the pharmaceutical composition can be used in treating disorders associated with glucagon.

Also within the present disclosure is a method for reducing the glycemic level in a subject, comprising administering to the subject in need thereof the aforesaid pharmaceutical composition of the present disclosure.

Further covered by the present disclosure is a method of treating disorders associated with glucagon, comprising administering to a subject in need thereof the aforesaid pharmaceutical composition of the present disclosure.

In the present disclosure, the aforesaid subject may be mammal, for example, a human, a pig, a horse, a cow, a dog, a cat, a mouse or a rat.

In the present disclosure, the diseases, conditions or disorders associated with glucagon can be, for example, hyperglycemia, Type II diabetes, metabolic syndrome, impaired glucose tolerance, glucosuria, diabetic nephropathy, diabetic neuropathy, diabetic retinopathy, hyperinsulinemia, insulin resistance syndrome, cataracts, obesity, dyslididemia, hypertension and myocardial infarction. However, the present disclosure is not limited thereto, and the pharmaceutical composition of the present disclosure can be applied to any other diseases, conditions or disorders associated with the glucagon signaling pathway. In one aspect of the present disclsoure, the diseases, conditions or disorders associated with glucagon may be hyperglycemia, Type II diabetes, impaired glucose tolerance, insulin resistance syndrome or obesity. In another aspect of the present disclosure, the diseases, conditions or disorders associated with glucagon may be Type II diabetes.

The following embodiments are made to clearly exhibit the above-mentioned and other technical contents, features and/or effects of the present disclosure. Through the exposition by means of the specific embodiments, people would further understand the technical means and effects the present disclosure adopts to achieve the above-indicated objectives. Moreover, as the contents disclosed herein should be readily understood and can be implemented by a person skilled in the art, all equivalent changes or modifications which do not depart from the concept of the present disclosure should be encompassed by the appended claims.

Moreover, in the present specification, a value may be interpreted to cover a range within ±10% of the value, and in particular, a range within ±5% of the value, except otherwise specified; a range may be interpreted to be composed of a plurality of subranges defined by a smaller endpoint, a smaller quartile, a median, a greater quartile, and a greater endpoint, except otherwise specified.

### EXAMPLE

Without further elaboration, it is believed that one skilled in the art can, based on the above description, utilize the present disclosure to its fullest extent. The following specific examples are therefore to be construed as merely illustrative, and not limitative of the remainder of the disclosure in any way whatsoever. All publications cited herein are incorporated by reference in their entirety.

The present disclosure is explained by the following embodiments, which are not used to limit the scope of the present disclosure. Unless specified otherwise, "%" used herein for indicating the amount of the contents or the objects in the following embodiments are weight percentage.

### Chemicals

ALP001E: The preparation of ALP001E can be referred to US 20190345118.
Acetone: It is used as a solvent.
Ethyl alcohol (200 Proof): It is used as a solvent.
Hypromellose Acetate Succinate: It is used as a dispersant or as a carrier in a solid dispersion of ALP001E.
Macrogol 15 Hydroxystearate: It not only improves the drug solubility and absorption, but also is associated with product stability. It is a mixture of mainly monoesters and diesters of 12-hydroxystearic acid and macrogols obtained by the ethoxylation of 12-hydroxystearic acid. It is also known as 12-hydroxyoctadecanoic acid polymer with α-hydro-ω-hydroxypoly(oxy-1,2-ethanediyl); 12-hydroxystearic acid polyethylene glycol copolymer; macrogol 15 hydroxystearate; polyethylene glycol-15-hydroxystearate; and polyethylene glycol 660 12-hydroxystearate.

### < Preparation of Formulations A to H>

The components of Formulations A to H are listed in the following Table 1. The parameters of the manufacture process of spray drying for Formulations A to H are listed in the following Table 2.

The steps for preparing the spray dry powders of Formulations A to H are briefly described below.

Step 1: Dissolving hypromellose acetate succinate into acetone and stirring the solution until hypromellose acetate succinate was completely dissolved as a clear or turbid viscous solution. The amounts of hypromellose acetate succinate and acetone are listed in Table 1.

Step 2: Dissolving Macrogol 15 Hydroxystearate into Ethyl Alcohol 200 Proof. The solution B was prepared while Macrogol 15 Hydroxystearate was completely dissolved into EtOH. The recipe of the solution B is also listed in Table 1.

Step 3: Rapidly dissolving ALP001E into the clear or turbid viscous solution prepared in Step 1 and stirring ALP001E and the solution until presenting as a clear or turbid viscous solution (solution A).

Step 4: Mixing the solution A and the solution B in a beaker and keeping stirring in the blender.

Step 5: Injecting the mixed solution into the spray dryer. The parameters of spray dryer are described as Table 2 and the feeding gas was nitrogen. The inlet temperature was adjusted in the range from 70 to 85°C until the outlet temperature was in the range from 42 to 52°C.

Step 6: Placing collected spray dry powders into the drying box for overnight (18-24 hr). This process is to remove the residue solvents to lower than 5000 ppm. The final product was stored in a glass bottle and kept in a refrigerator at -30°C. ALP001E and the final products may be unstable under high moisture condition. Therefore, the protocol should be executed in moisture control environment.

**Table 1**

| | Formulation A | | Formulation B | |
|---|---|---|---|---|
| Component | Amount | Percentage | Amount | Percentage |
| ALP001E | 10 mg | 22.4% | 10 mg | 18.3% |
| Hypromellose Acetate Succinate | 30 mg | 67.3% | 40 mg | 73.3% |
| Macrogol 15 Hydroxystearate | 4.6 mg | 10.3% | 4.6 mg | 8.4% |
| Total | 44.6mg | 100% | 54.6mg | 100% |
| Acetone^{∗} | 1600 mg | - | 800 mg | - |
| Ethyl Alcohol 200 Proof^{∗} | 20 mg | - | 20 mg | - |

| | Formulation C | | Formulation D | |
|---|---|---|---|---|
| Component | Amount | Percentage | Amount | Percentage |
| ALP001E | 10 mg | 26.3% | 10 mg | 30.1% |
| Hypromellose Acetate Succinate | 23.4 mg | 61.6% | 18.6 mg | 56.0% |
| Macrogol 15 Hydroxystearate | 4.6 mg | 12.1% | 4.6 mg | 13.9% |
| Total | 38mg | 100% | 33.2mg | 100% |
| Acetone^{∗} | 1340 mg | - | 1150 mg | - |
| Ethyl Alcohol 200 Proof^{∗} | 20 mg | - | 20 mg | - |

| | Formulation E | | Formulation F | |
|---|---|---|---|---|
| Component | Amount | Percentage | Amount | Percentage |
| ALP001E | 10.0 mg | 15.8% | 10.0 mg | 9.6% |
| Hypromellose Acetate Succinate | 48.8 mg | 77.0% | 90.0 mg | 86.0% |
| Macrogol 15 Hydroxystearate | 4.6 mg | 7.3% | 4.6 mg | 4.4% |
| Total | 63.4mg | 100% | 104.6mg | 100% |
| Acetone^{∗} | 1306.0 mg | - | 2220.0 mg | - |
| Ethyl Alcohol 200 Proof^{∗} | 20.0 mg | - | 20.0 mg | - |

| | Formulation G | | Formulation H | |
|---|---|---|---|---|
| Component | Amount | Percentage | Amount | Percentage |
| ALP001E | 10mg | 14% | 10mg | 33.8% |
| Hypromellose Acetate Succinate | 56.7mg | 79.5% | 15mg | 50.7% |
| Macrogol 15 Hydroxystearate | 4.6mg | 6.5% | 4.6mg | 15.5% |
| Total | 71.3mg | 100% | 29.6mg | 100% |
| Acetone^{∗} | 1067.2 mg | - | 1000mg | - |
| Ethyl Alcohol 200 Proof^{∗} | 20.0 mg | - | 20mg | - |

| | | | | |
|---|---|---|---|---|
| ^{∗}To be evaporated during the drying process. | | | | |

**Table 2**

| | Formulation A | Formulation B | Formulation C | Formulation D |
|---|---|---|---|---|
| Spray nozzle orifice size | 1A (Standard) (Particle size: 1~40 µm) | | | |
| Inlet temperature | 70~85°C | 70~85°C | 70~85°C | 70~85°C |
| Outlet temperature | 42~52°C | 42~52°C | 42~52°C | 42~52°C |
| Pump speed of solution | 3~4 (3.5) | 3~4 (3.5) | 3 | 3 |
| Atomization pressure | 0.20~0.22 Mpa | 0.15∼0.20 Mpa | 0.20~0.22 Mpa | 0.20~0.22 Mpa |
| Blower | Scale 7 | Scale 7 | Scale 5 | Scale 5 |
| Air volume | 0.40 m³/min | 0.40 m³/min | 0.40 m³/min | 0.40 m³/min |
| Oxygen content (%) | <3% | <3% | <3% | <3% |

| | Formulation E | Formulation F | Formulation G | Formulation H |
|---|---|---|---|---|
| Spray nozzle orifice size | 1A (Standard) (Particle size: 1~40 µm) | | | |
| Inlet temperature | 70~85°C | 70~85°C | 70~85°C | 70~85°C |
| Outlet temperature | 42~52°C | 42~52°C | 42~52°C | 42~52°C |
| Pump speed of solution | 3 | 3 | 3 | 3 |
| Atomization pressure | 0.20~0.22 Mpa | 0.20~0.22 Mpa | 0.20~0.22 Mpa | 0.20~0.22 Mpa |
| Blower | Scale 6 | Scale 6 | Scale 6 | Scale 6 |
| Air volume | 0.40 m³/min | 0.40 m³/min | 0.40 m³/min | 0.40 m³/min |
| Oxygen content (%) | <3% | <3% | <3% | <3% |

### < Property measurment>

The properties of the obtained powders of Formulations A to H were measured, and the results are listed in the following Table 3. The measurement methods are briefly described below.

In the present example, all of the ingredients of the formulations were measured by the use of the electronic balance (M Mettler-Toledo Basic Moisture Analyzer) and converted to yield by calculation. The appearances of the formulations were check by the visual inspection.

To measure the degradation products from the formulations were using a HPLC system. The HPLC analysis was performed using an Agilent 1100 HPLC system comprising a quaternary pump, an in-line degasser, an auto-sampler and a photodiode array detector. The column employed was a Thermo Scientific Hypersil BDS C18 reverse phase column, 250 mm x 4.6 mm, 5 µm.

A Metrohm Coulometer model 917 with Compact oven SC 885 auto sampler was used for the analysis. 50 mg of the powder sample was tested in triplicate, and the oven temperature was set to 105°C. The reagent used for analysis was hydranal Coulomat AG Oven.

Water content was also analysed using an AND MX-50 moisture balance. Approximately 1 g of material was placed on a metal pan and heated to 105°C until the weight change is less than 0.01%.

DSC analysis was undertaken using a TA Instruments Q20 MDSC with auto sampler and refrigerated cooling accessory. Approximately 1-5 mg of sample was sealed in a TZero aluminium pan using a TZero pan press.

To measure the mean particle size are using a powder particle size distribution instrument (Syepatec HELOS particle size analyzer).

**Table 3**

| | Formulation A | Formulation B | Formulation C | Formulation D |
|---|---|---|---|---|
| Yield (%) | 50~75% | 75~95% | 50~75% | 50~75% |
| Appearance | No aggregation | No aggregation | Aggregation, poor dispersion | Aggregation, poor dispersion |
| Degradation products (HPLC)¹ | individual degradation NMT <0.1% | individual degradation NMT <0.1% | individual degradation NMT <0.1% | individual degradation NMT <0.1% |
| Water content (%) | <6% | <3% | <6% | <6% |
| DSC¹ | Amorphous | Amorphous | Amorphous | Tₘ peak of ALP001E peak |
| Mean particle size (µm) | 8.02 | 15.22 | 30.41 | ND |
| | Formulation E | Formulation F | Formulation G | Formulation H |
| Yield (%) | 50~75% | 50~75% | 50~75% | 50~75% |
| Appearance | No aggregation | No aggregation | No aggregation | Aggregation, poor dispersion |
| Degradation products (HPLC)¹ | individual degradation NMT <0.1% | individual degradation NMT <0.1% | individual degradation NMT <0.1% | individual degradation NMT <0.1% |
| Water content (%) | <3% | <6% | <6% | <6% |
| DSC¹ | Amorphous | Amorphous | Amorphous | Tₘ peak of ALP001E peak |
| Mean particle size (µm) | 9.22 | ND | ND | ND |

| | | | | |
|---|---|---|---|---|
| 1: The dry powders of Formulations A to H were placed at 60°C for 7 days, and these data indicated the thermal stability of Formulations A to H. | | | | |

The appearance, the impurity and the thermal stability of the dry powders of Fomulations A to H were measured by placing the dry powders of Fomulations A to H at 60°C for 7 days (Day 7) and compared with the fresh dry powders of Fomulations A to H (Day 0). From the results shown in Table 3, no significant appearance changes were observed in the dry powders of Formulations A, B, and E to G at Day 7 compared with the dry powders of Formulations A, B, and E to G at Day 0. In addition, the HPLC data indicate that no more than 0.1% impuratiy was observed in the dry powders of Formulas A to H at Day 7.

Furthermore, the DSC data of the dry powders of Formulations A to C and E to G show no significant change at Day 7 compared to the dry powders of Formulations A to C and E to G at Day 0, but Tₘ peak of ALP001E peak (Tₘ range: from 120°C to 125°C) between 115°C and 130°C was observed in the dry powders of Formulations D and H at Day 7 compared to the dry powders of Formulations D and H at Day 0. These results indicate the dry powders of Formulations A to C and E to G have excellent stability.

Furthermore, as shown in Table 3, the ranges of particle distributions of the dry powders of Formulations A to C and E are not the same. In the dry powders of Fomulation A, D10 (Dv 0.1) is 1~3 µm, D50 (Dv 0.5) is 4~10 µm, and D90 (Dv 0.9) is 11~20 µm. In the dry powders of Formulation B, D10 (Dv 0.1) is 1~3 µm, D50 (Dv 0.5) is 5~15 µm, and D90 (Dv 0.9) is 25~50 µm. In the dry powders of Formulation C, D10 (Dv 0.1) is 3~6 µm, D50 (Dv 0.5) is 20~35 µm, and D90 (Dv 0.9) is 50~70 µm. In the dry powders of Formulation E, D10 (Dv 0.1) is 1~3 µm, D50 (Dv 0.5) is 4~10 µm, and D90 (Dv 0.9) is 11~20 µm. Herein, D50 (Dv 0.5) is the particle size of the median for a volume distribution; and D10 (Dv 0.1) and D90 (Dv 0.9) respectively the particle size below which 10% and 90% of the volume of particles exists.

### < In vitro dissolution testing>

*In vitro* dissolution testing was performed using an USP dissolution apparatus Type 2 (paddles) at 75 rpm (250 rpm at infinity) using 900 mL phosphate buffer pH 6.8 with 0.25% w/v SDS as the dissolution medium at 37 ± 0.5°C. The parameters for the *in vitro* dissolution testing are listed in the following Table 4, and the results are listed in the following Table 5.

**Table 4**

| USP Apparatus | II (Paddle) |
|---|---|
| Speed (RPMs) | 75 |
| Medium | 0.05 M Sodium Phosphate Buffer with 0.25% Sodium Dodecyl Sulfate, pH 6.8 |
| Volume (mL) | 900 |
| Temperature (°C) | 37 ± 0.5 |
| Sampling Times (minutes) | 5, 10, 15, 20, 30, 45, 60, 90 & 120 |
| Standard Solution | 0.0444 mg/mL of APL001E |

**Table 5**

| Time (min) | ALP001E | Formulation | | | | | |
|---|---|---|---|---|---|---|---|
| | | A | B | C | E | F | G |
| 0 | 0.00% | 0.00% | 0.00% | 0.00% | 0.00% | 0.00% | 0.00% |
| 5 | 4.40% | 9.17% | 9.87% | 5.56% | 7.49% | 1.17% | 0.00% |
| 10 | 3.69% | 25.27% | 23.20% | 12.79% | 21.83% | 7.34% | 5.81% |
| 15 | 4.73% | 42.50% | 39.24% | 21.74% | 34.81% | 12.84% | 13.14% |
| 20 | 2.87% | 57.09% | 41.23% | 34.13% | 47.90% | 18.32% | 19.36% |
| 30 | 1.18% | 74.61% | 66.14% | 52.82% | 62.55% | 26.99% | 33.05% |
| 45 | 1.26% | 90.60% | 100.18% | 77.94% | 75.44% | 37.85% | 45.32% |
| 60 | 2.11% | 97.27% | 100.08% | 93.62% | 84.61% | 46.09% | 55.94% |
| 90 | 2.77% | 98.61% | 104.56% | 97.11% | 93.57% | 61.65% | 73.88% |
| 120 | 13.04% | 98.97% | 102.58% | 97.39% | 97.70% | 74.91% | 89.19% |

According to the reulsts shown in Table 5, the solubility of the dry powders of Formulations A to C and E to G are significantly increased compared with the solubility of APL001E, and in particular, the solubility of the dry powders of Formulations A to C and E to G are more significantly increased. These data indicate that the dry powders of Formulations A to C and E to G have improved solubility.

### <Solubility>

The method for measuring the solubilities of the formulations is briefly described.

The test formulation was added into indicated buffers or solutions (ddH₂O or 0.9% saline, pH 7.4) to the final concentration of 3 mg/mL, followed by sonicating for 30 mins. Next, the mixture was put into 37°C water bath for 24 hrs. The mixture was mixed well by vortex and 1 mL of mixture was transferred into the 1.5 mL eppendorf tube, followed by centrifuging at 20817 xg for 30 mins. 10 µL of the supernatant was collected, and the concentration of the collected supernatant was determined by LC-MS/MS. The results are listed in the following Table 6.

**Table 6**

| | Solubility (mg/ml) | | |
|---|---|---|---|
| | USP pH 6.8 | H₂O (pH7.0) | Saline (pH7.4) |
| ALP001E | 4.40E-04 | < LOQ | < LOQ |
| Formulation A | 5.29E-02 | 3.95E-03 | 4.02E-03 |
| Formulation B | 4.72E-02 | 1.74E-03 | 1.65E-03 |
| Formulation C | 9.19E-02 | 3.64E-03 | 4.84E-03 |
| Formulation E | 2.03E-02 | 1.73E-03 | 2.47E-03 |
| Formulation F | 2.62E-02 | 2.44E-03 | 1.92E-03 |
| Formulation G | 2.38E-02 | 2.58E-03 | 1.76E-03 |

According to the reulsts shown in Table 6, the solubility of the dry powders of Formulations A to C and E to G are significantly increased compared with the solubility of APL001E. These data indicate that the dry powders of Formulations A to C and E to G have improved solubility.

### <Oral bioavailability >

Briefly, three healthy, research Beagle dogs, aged 4~10 years and body weight 9~12 kg were used. The pharmacokinetics experiments have intravenous bolus (IV bolus) and oral groups.

For the IV bolus group, ALP001 (an active metabolite compound from ALP001E, represented by the following formula (II)) dissolved in vehicle (0.1M NaHCO₃, pH7.4) was administered to the dogs via IV bolus with a dose strength ranging from 40 to 50 mg depending the weight of dog on the experiment design. Base on the non-compartmental analysis to obtain pharmacokinetics parameters, the AUC (area under the curve) of IV bolus is 2.18 µg.h/mL. It will be used in bioavailability estimation.

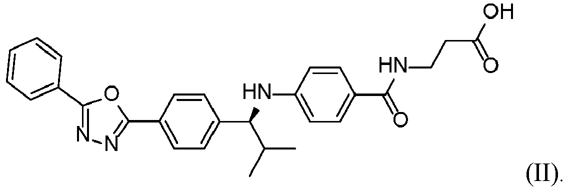

For the oral group, the capsule comprising dry powders of formulations was orally administered to the dogs. The dogs were weighted and the blood of the dogs was taken before the capsule was administered. The capsule comprising spray drying powders was stuffed into the dog's mouth (wherein the dose of the active ingredient ALP001E is ranged from 35 mg to 50 mg, depending on the weight of animal in experiment design) and 3 ml water was given immediately after the capsule stuffed into the dog's mouth.

For the IV bolus and oral groups, the dogs received the drugs after fasting for 4 hours, and the blood samples of the dogs were taken before administration. The blood samples were collected at pre-assigned times (10 min, 1 hr, 2 hr, 4 hr, 5 hr, 7 hr and 24 hr) after administration. The concentrations of the drug in plasma were determined with validated high performance liquid chromatography.

The plasma concentration data were analyzed and modeled by the non-compartmental analysis to obtain pharmacokinetics parameters. In addition, the bioavailability was obtained by comparing the data of the oral group with the data of the IV bolus group. The results are listed in the following Table 7.

**Table 7**

| Formulation | Oral AUCₐₗₗ (µg.h/mL) | Bioavailability (%) |
|---|---|---|
| ALP001E (No formulation in API) | 0.08 | 3.7% |
| A | 0.78 | 35.8% |
| B | 1.27 | 58.4% |
| C | 0.54 | 24.5% |
| E | 1.17 | 54.6% |
| G | 0.52 | 23.9% |

The results shown in Table 7 indicate that the oral dry powders of Formulations A to C, E and G, in particular the oral dry powders of Formulations B and E, have good bioavailability. In addition, the oral bioavailability of dry powders is increased when the ratio of hypromellose acetate succinate to Macrogol 15 Hydroxystearate is within a desired range. In particular, the oral bioavailabilities of the dry powders of Formulations B and E are respectively 58.4% and 54.6%.

From the results shown above, the present disclosure provides a novel pharmaceuticaly composition, which has improved stability, acceptable solubility or increased oral absorption. Thus, the pharmaceutical composition of the prenset disclosure can be used for reducing the glycemic level or treating disorders associated with glucagon in a subject thereof by oral administration.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

Further, from the above description, one skilled in the art can easily ascertain the essential characteristics of the present disclosure, and without departing from the spirit and scope thereof, can make various changes and modifications of the disclosure to adapt it to various usages and conditions. Thus, other embodiments are also within the claims.

## Claims

1. A pharmaceutical composition, comprising:
an active ingredient of the following formula (I) or a pharmaceutically acceptable salt or solvate thereof present in an amount of 8 wt% to 30 wt%: 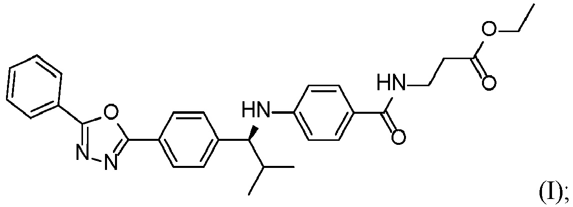
two or more excipients at least including a dispersant and a solubilizer present in an amount of 70 wt% to 92 wt%.

2. The pharmaceutical composition of claim 1, wherein a weight ratio of the dispersant to the solubilizer is ranged from 3.0 to 20.0.

3. The pharmaceutical composition of claim 1 or 2, wherein the active ingredient of the formula (I) or the pharmaceutically acceptable salt or solvate thereof is present in an amount of 9 wt% to 28 wt%, in an amount of 10 wt% to 28 wt%, or in an amount of 14.5 wt% to 20 wt%.

4. The pharmaceutical composition of any of the previous claims, wherein the dispersant is present in an amount of 40 wt% to 88 wt%, in an amount of 60 wt% to 85 wt%, or in an amount of 70 wt% to 80 wt%.

5. The pharmaceutical composition of any of the previous claims, wherein the solubilizer is present in an amount of 2 wt% to 20 wt%, in an amount of 5 wt% to 13 wt%; or in an amount of 5 wt% to 10 wt%.

6. The pharmaceutical composition of any of the previous claims, which is an oral pharmaceutical composition.

7. The pharmaceutical composition of any of the previous claims, wherein the dispersant comprises hypromellose acetate succinate.

8. The pharmaceutical composition of any of the previous claims, wherein the solubilizer comprises Macrogol 15 Hydroxystearate.

9. The pharmaceutical composition of any of the previous claims, wherein the active ingredient of the formula (I) or the pharmaceutically acceptable salt or solvate thereof is present in an amount of 10 wt% to 28 wt%; the dispersant is present in an amount of 60 wt% to 85 wt%; and the solubilizer is present in an amount of 5 wt% to 13 wt%.

10. The pharmaceutical composition of claim 9, wherein the dispersant comprises hypromellose acetate succinate and the solubilizer comprises Macrogol 15 Hydroxystearate.

11. The pharmaceutical composition of any of the previous claims, wherein the active ingredient of the formula (I) or the pharmaceutically acceptable salt or solvate thereof is present in an amount of 14.5 wt% to 20 wt%; the dispersant is present in an amount of 70 wt% to 80 wt%; and the solubilizer is present in an amount of 5 wt% to 10 wt%.

12. The pharmaceutical composition of claim 11, wherein the dispersant comprises hypromellose acetate succinate and the solubilizer comprises Macrogol 15 Hydroxystearate.

13. Pharmaceutical composition for use in a method for reducing the glycemic level in a subject, said method comprising administering to the subject in need thereof said pharmaceutical composition, wherein said pharmaceutical composition comprises:
an active ingredient of the following formula (I) or a pharmaceutically acceptable salt or solvate thereof present in an amount of 8 wt% to 30 wt%: 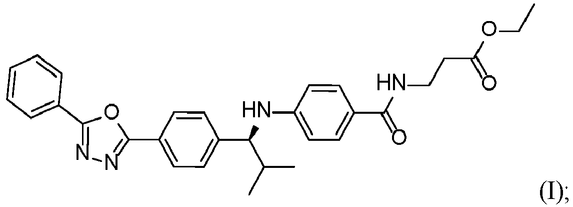 and
two or more excipients at least including a dispersant and a solubilizer present in an amount of 70 wt% to 92 wt%.

14. Pharmaceutical composition for use in a method of treating disorders associated with glucagon, said method comprising administering to a subject in need thereof said pharmaceutical composition, wherein said pharmaceutical composition comprises:
an active ingredient of the following formula (I) or a pharmaceutically acceptable salt or solvate thereof present in an amount of 8 wt% to 30 wt%: 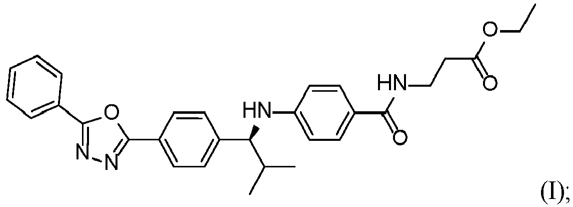 and
two or more excipients at least including a dispersant and a solubilizer present in an amount of 70 wt% to 92 wt%.
